Europäisches Patentamt

European Patent Office

Office européen des brevets

(19)

(11) Publication number: **0 302 300 B1**

# EUROPEAN PATENT SPECIFICATION

(12)

(45) Date of publication of patent specification: **21.04.93**

(51) Int. Cl.⁵: **C12P 1/04**, C12N 1/20, A23L 3/34, A23B 7/10, A23K 3/00, //(C12N1/20, C12R1:245)

(21) Application number: **88111628.9**

(22) Date of filing: **19.07.88**

(54) **Process for producing novel yeast and mold inhibiting products.**

(30) Priority: **06.08.87 US 82118**

(43) Date of publication of application:
**08.02.89 Bulletin 89/06**

(45) Publication of the grant of the patent:
**21.04.93 Bulletin 93/16**

(84) Designated Contracting States:
**AT BE CH DE ES FR GB GR IT LI LU NL SE**

(56) References cited:
**EP-A- 0 221 499**

**DIFCO Manual, 10th edition, pp. 1089-1090**

(73) Proprietor: **MICROLIFE TECHNICS, INC.**
**1833 57th Street**
**Sarasota Florida 33578(US)**

(72) Inventor: **Vandenbergh, Peter A.**
**4414 Meadowcreek Circle**
**Sarasota Florida 33583(US)**
Inventor: **King, Stephen W.**
**675 Lathrop Court**
**Napa California 94558(US)**

(74) Representative: **Patentanwälte Grünecker,**
**Kinkeldey, Stockmair & Partner**
**Maximilianstrasse 58**
**W-8000 München 22 (DE)**

EP 0 302 300 B1

**Description**

The present invention relates to yeast and mold (fungus) inhibiting products produced by Lactobacillus species. In particular, the present invention relates to a process for producing the products and to their use in preventing yeast and mold growth in foods and other materials.

Various substances are produced by microorganisms which are antimicrobial in character. Lactobacillus are known to produce metabolic products that are antibacterial and allow them to compete more effectively in certain environments. In EP-A-0 221 499 a method is described for providing fungal inhibition using Lactobacillus casei var. rhamnosus which produces an antifungal substance.

It is therefore an object of the present invention to provide novel Lactobacillus metabolic products which inhibit yeast and mold and which are referred to herein as "FIC" or fungal inhibiting compounds. Further it is an object of the present invention to provide a process for producing the FIC products as well as a method for using these FIC products in foods and other materials. These and other objects will become increasingly apparent by reference to the following description.

The present invention relates to a process for producing yeast and mold inhibiting products (FIC) which comprises: incubating live cells of a Lactobacillus species in a nutrient medium for the cells including a sulfur containing organic compound (preferably cysteine, garlic extract, whey, yeast, yeast extract, molasses or protein digest) which induces the formation of the products (FIC), a protein source, and a carbon source, so as to produce an isolatable amount of the products (FIC) in the nutrient medium, wherein the products (FIC) inhibit Penicillium oxalicum spores in an assay with the products (FIC) and the Penicillium oxalicum spores mixed.

The present invention relates to a process for producing yeast and mold inhibiting products (FIC) which comprises: incubating live cells of a Lactobacillus species in a nutrient medium containing growth factors present in cysteine, garlic extract, milk, whey, yeast, yeast extract, molasses or protein digest which induce the formation of the products (FIC), a protein source and a carbon source, so as to produce the products (FIC) in the nutrient medium; and treating the nutrient medium which has been incubated so as to produce the products (FIC) with or without the live cells, wherein the products (FIC) inhibit Penicillium oxalicum spores in an assay with the products (FIC) and the Penicillium oxalicum spores mixed.

Further still the present invention relates to a process for producing yeast and mold inhibiting products (FIC) which comprises: incubating live cells of a Lactobacillus in a nutrient medium for the cells containing growth factors present in cysteine, garlic extract, milk, whey, yeast, yeast extract, molasses or protein digest which induce the formation of the products (FIC), and a carbon source so as to produce products (FIC) in the nutrient medium wherein the products (FIC) inhibit Penicillium oxalicum spores in an assay with the products (FIC) and the Penicillium oxalicum spores mixed; and treating the nutrient medium which has been incubated so as to produce the products (FIC) with the cells disrupted.

Finally the present invention relates to a method for preventing yeast and mold growth in a material in need thereof which comprises: adding to the material products (FIC) produced by a process which comprises incubating live cells of a Lactobacillus in a nutrient medium containing a sulfur containing organic compound, a protein source and a carbon source which induce the formation of the yeast and mold inhibiting products (FIC) in the nutrient medium wherein the products (FIC) inhibit Pencillium oxalicum spores in an assay with the products (FIC) and the Penicillium oxalicum spores mixed and then treating the nutrient medium which now contains live cells, in such a manner so as to produce the final products (FIC) in a form containing live cells, dead cells or no cells to thereby prevent yeast and mold growth in the material.

The preferred products (FIC) from Lactobacillus casei var. rhamnosus NRRL-B-15972 have a molecular size of less than 1000 daltons and an ultraviolet absorbance at 269 nanometers. It is a complex mixture of compounds.

Lactobacillus casei var. rhamnosus NRRL-B-15972 is described in application Serial No. 794,468. It is deposited with the Northern Regional Research Laboratory in Peoria, Illinois. Any Lactobacillus strain, whether naturally occurring or genetically engineered, with genetic material (in chromosomes or plasmids) encoding for similar yeast and mold inhibiting products (FIC) can be used to produce the products (FIC), although Lactobacillus casei var. rhamnosus NRRL-B-15972 appears to be the most effective naturally occurring (non-genetically engineered) source.

Various growth media for Lactobacillus can be used. The media must promote growth of cells and production of the products (FIC). Media to promote growth must contain protein and a carbon source. In addition, for products (FIC) production the media preferably includes a sulfur containing organic compound. Preferably the growth media include a sulfur containing organic compound, a protein source, a carbon source and minerals. Proteinaceous or amino acid materials from natural sources can include the sulfur containing compound. Thus protein sources such as milk, whey, yeast, yeast extract or protein digest can be a source of the sulfur compound and stimulate the production of the products (FIC). Separate compounds containing sulfur without being a protein source, such as garlic extract, molasses and cysteine, can be used. The carbon source can include fructose, sucrose, dextrose, lactose or molasses. Minerals which facilitate growth of the cells, such as manganese and magnesium salts are available in corn steep liquor or can be added as pure salts. Preferably buffers such as alkali metal phosphates are used to maintain the pH. It has been found that cinnamic acid and/or an alkali metal propionate and/or phenylalanine further stimulate the production of the yeast and mold inhibitory products (FIC) in the above referenced media. Preferably the cells are grown at a temperature between 10° and 50°C. Numerous variations of the nutrient medium and growth conditions will occur to those skilled in the art.

After growth of the cells, the nutrient medium preferably is processed to eliminate most of the live cells and then either concentrated or extracted to produce the yeast and mold inhibiting products (FIC). The yeast and mold inhibiting products (FIC) can be dried, lyophilized or frozen prior to use. The preferred process for producing the products (FIC) in a relatively unconcentrated form is spray drying the growth medium after the yeast and mold inhibitory products are produced in the medium. All of these methods are well known to those skilled in the art.

Various water immiscible solvents can be used to extract the products from the growth medium, such as lower alkyl alcohols and esters. Preferably n-butanol isopropanol, acetone, ethyl acetate or ethanol and combinations with water are used. The products can also be separated by chromatographic methods including molecular sieve, ion exchange and high pressure liquid chromatography methods well known to those skilled in the art. Impurities can be removed from the products (FIC) using molecular sieves and reverse osmosis. Essentially any chemical and/or mechanical process can be used for the separation.

As used herein, the term "material" means any surface in need of treatment by the FIC. The term material includes living and non-living surfaces. The yeast and mold inhibiting products (FIC) are preferably used in foods in an amount which inhibits P. oxalicum for at least 72 hours. The amount of FIC used depends upon the number of yeast and mold cells in the material to be treated.

Any food can be preserved by the method of the present invention especially carbonated beverages, Cottage cheese, yogurt, margarine, bread, grains and nuts. Because the products (FIC) are effective in a broad pH range pH 3 to 10 they are especially useful for use to preserve any food by the method of the present invention. The yeast and mold inhibiting products (FIC) are particularly useful in fermented foods and other preserved foods which are prone to such spoilage. Other food applications include silage and corn mold treatment and peanut treatment to prevent aflatoxin contamination by mold. Various materials can be treated and FIC can be used to prevent infection by mold and yeast, including living tissue as the material either in culture or in an animal, particularly mammals. Topical application of FIC to mammals either internal or external is preferred. Detergents, soaps and other cleansers can be combined with the products (FIC).

Lactobacillus casei var. rhamnosus NRRL-B-15972 produces products (FIC) which inhibit a wide variety of yeasts including: P. camemberti, B. allii, Caldosporium, Debaromyces sp., S. cerevisiae (Baker's) and molds. The compounds in the products (FIC) are polar and have a molecular size of less than 1,000 daltons and are not proteins, or lipids. The product (FIC) is unique in that it is insoluble in chloroform and n-hexane, and soluble in ethanol, n-butanol and acetone. The FIC appears to be temperature stable, i.e. -70°C to $^+$100°C, however it is destroyed by autoclaving at 121°C. FIC is stable from pH 3-10. Nutritional studies indicate that alkali metal propionate, phenylalanine, and/or cinnamic acid appear to further stimulate production of the yeast and mold inhibiting products by Lactobacillus casei var. rhamnosus as well as by other Lactobacilli. Purification of the products (FIC) can be obtained through the use of organic extraction and flash evaporation. The following examples show the method of production and use of the yeast and mold inhibiting products (FIC) of the present invention.

Example 1

Lactobacillus sp. Exhibiting Antifungal Activity.

Antifungal products (FIC) are produced by Lactobacillus having various degrees of activity. These are shown in Table 1 wherein various cultures grown on agar were tested against P. oxalicum spores.

Table 1

| | Fic Zone 48h[1] |
|---|---|
| Lactobacillus casei var. rhamnosus NRRL-B-15972 | 5.0mm |
| L. casei var. casei 2610 | 3.0mm |
| L. casei var. casei 2601 | 4.0mm |
| L. casei var. tolerans | 4.0mm |
| L. casei VH | 4.0mm |
| L. plantarum ATCC 8014 | 4.0mm |
| L. acidophilus SFS[2] | No Zone Produced |
| L. bulgaricus YB-1[2] | 2.0mm |
| L. bulgaricus DFW[2] | 3.0mm |
| L. bulgaricus LBHW[2] | 3.0mm |
| L. bulgaricus HCOYWC[2] | 3.0mm |
| L. parma | No Zone Produced |

(1) The Cells were grown 24h at 35°C on MRS agar medium (DIFCO, Detroit, Michigan). MRS™ contains yeast extract along with proteose peptone, beef extract, sodium acetate, sodium citrate and dextrose. Agar is added to form a gel. The live cells were then overlayed on the agar with $10^6$ spores/ml of the fungus Penicillium oxalicum. The width of the clear zone was measured.

(2) These cultures were grown in a confined chamber at 35°C with carbon dioxide and then challenged with the P. oxalicum. The L. bulgaricus and L. casei strains listed in Table 1 have different genetic and physiological characteristics. All of the strains are on deposit at Microlife Technics, Inc., Sarasota, Florida.

None of the Lactobacilli were as effective as L. casei var. rhamnosus NRRL-B-15972. This strain was used for the subsequent Examples.

Example 2

Preparation, production and purification of liquid yeast and mold inhibiting products (FIC).

Medium Incubation - A culture of Lactobacillus casei var. rhamnosus NRRL-B-15972 was grown in MRS™ broth (Difco, Detroit, Michigan) supplemented with 1% by weight yeast extract (Oxoid®, Oxoid Ltd., Basingstoke, Hampshire, England). MRS™ contains yeast extract, proteose peptone, beef extract, sodium acetate, sodium citrate, and dextrose in a broth. One (1) liter of the medium was sterilized, inoculated with the culture and incubated at 35°C without shaking or neutralization for 18 hours.

The purification procedure was:

1. Flash Evaporation - The cells and medium were concentrated (1 liter to 100 ml) using a flash evaporator (Rotor-Vap™). A dark brown mixture remained (100 ml).

2. Butanol - The concentrated cells-medium suspension was then mixed with 1 liter of n-butanol. This mixture was placed in a separatory funnel and separation occurred in 30 minutes. The bottom water layer was discarded. The upper layer (n-butanol layer) was then concentrated using the flash evaporator until a dry particulate material remained. This material was then resuspended in 50 ml of distilled water.

3. Ethanol Extraction - To the 50 ml of distilled water mixture, 250 ml of ethanol (100%) was added and impurities were allowed to precipitate overnight at -70°C. After precipitation had occurred, the solution was centrifuged at -20°C for 20 minutes at 12,000 x g. The supernatant was then concentrated on the flash evaporator. The oily material was resuspended in 10 to 20 ml of distilled water.

4. Column Chromatography - A Pharmacia column (2.6 cm x 35 cm) containing 125 g of silica gel (Sigma™, St. Louis Missouri). 28-200 mesh, 22 Angstrom mean pore diameter) was prepared. The silica gel was suspended in methanol, chloroform and ethyl acetate in a ratio of 1:2:3 respectively. The total column volume was 186 ml. Approximately 5 ml of the concentrated ethanol fraction was loaded onto this column. The column was washed with approximately 400 ml of the above solvent. The column washings were discarded.

The column was then washed with a mixture of water, 5% ammonium hydroxide, glacial acetic acid, acetone and n-butanol as in a ratio of respectively 2:3:3:5:7 as a solvent. The solvent was collected in 5 ml fractions. The 20th through 30th fractions which contained the antifungal substance were saved. These fractions were collected and concentrated using the flash evaporator and then resuspended in 5

4

ml of distilled water.

5. Acetone Precipitation - To 5 ml of the concentrated column material, 150 ml of acetone was added and precipitation was allowed to occur overnight at - 70°C to remove impurities. The supernatant was centrifuged, the acetone was flash evaporated on the flash evaporator and the resulting antifungal substance was resuspended in 3 ml of distilled water.

FIC was assayed using a) MRS (Difco) agar plates; b) MRS (Difco) soft agar, (1 g of Bacto™ (Difco) agar per 100 ml MRS broth) at 8 ml/plate tempered at 55°C; c) Penicillium oxalicum spores $10^8$/ml in distilled water, at 1 ml per assay; and d) The material to be assayed.

Eight (8) ml of soft agar was combined with 1 ml of the spores ($10^8$/ml) and 1 ml of assay material, the mixture was vortexed and the contents were poured over an MRS plate and then incubated at 25°C. The plates then were examined for fungal growth. There is either growth or no growth on the plate. The degree of growth is rated between +1 and +4 as described hereinafter. The final purified product resulted in no growth of P. oxalicum after 96 hours.

Example 3

This example shows an alternate extraction method for producing an FIC similar to Example 2.

Medium and Incubation - The culture was grown in Folic Acid Assay Medium (DIFCO, Detroit, Michigan) supplemented with 1% yeast extract (OXOID®) or 1% whey protein concentrate or 1% garlic extract. One liter of the medium was sterilized, inoculated with culture and incubated at 35°C for 18 hours without shaking or neutralization.

The purification procedure was:

1. Butanol Extraction - The cells and medium suspension were mixed with 5 liters of n-butanol. The mixture was placed in a separatory funnel and the n-butanol layer was saved. This layer was then concentrated using the flash evaporator.

2. Ethanol Extraction - The above mixture (50 ml) was combined with 250 ml of ethanol (100%) and precipitated overnight at -70°C. The material was centrifuged at -20°C for 20 minutes at 12,000 x g. The supernatant was then decanted and concentrated using the flash evaporator.

3. Dialysis - The ethanol precipitate was then placed in a dialysis bag with 1000 dalton molecular size cut off. The bag and its contents were then dialyzed against distilled water (100 ml) at 4°C for 18 h. The distilled water was then concentrated on the flash evaporator.

FIC was assayed as in Example 2. The results are shown in Table 2.

Table 2*

| Growth Period | Folic Acid Assay Medium Unsupple-mented | Folic Acid Assay Medium plus 1% yeast extract | Folic Acid Assay Medium Plus 1% Whey Protein Concentrate | Folid Acid Assay Medium Plus 1% Garlic Extract |
|---|---|---|---|---|
| 24 h | +1 | - | - | - |
| 48 h | +2 | +1 | - | - |
| 96 h | +4 | +2 | +1 | - |

* assayed as in Example 2 with a spore concentration of $10^7$ spores per ml.

- = no mycelial masses present.

+1 = mycelial masses present, greater than 1000.

+2 = confluent white lawn.

Folic acid based assay medium supplemented with 1% garlic extract produced the highest concentration of FIC.

Example 4

Comparison of the Expression of FIC by L. casei var. rhamnosus NRRL-B-15972 In Various Nutrient Media.

Lactobacillus casei var. rhamnosus NRRL-B-15972 was inoculated into 250 ml of various nutrient media and incubated at 35°C for 18 hours. The cells and media were then extracted and processed as previously described in Example 2. One ml of extracted material was then assayed using the fungal biological method of Example 2, and with a spore concentration of $10^7$ spores per ml. The assay plates were examined for fungal growth after 24 hours, 48 hours and 72 hours incubation, respectively. The results are shown in Table 3.

Table 3

| Medium | Incubation Time | | |
| --- | --- | --- | --- |
| | 24h | 48h | 72h |
| Control uninoculated MRS broth (Difco®) supplemented with 1% yeast extract (Oxoid®). | +1 | +4 | +4 |
| MRS Broth (Difco®). | − | +2 | +4 |
| Whey based broth supplemented with 1% yeast extract (Oxoid®). | +1 | +4 | +4 |
| MRS broth (Difco®) supplemented with 1% yeast extract (Oxoid®) | − | − | +1 |
| MRS broth (Difco®) supplemented with 1% yeast extract (Tureen®) | − | +1 | +2 |
| MRS broth (Difco®) supplemented with 0.01% sodium propionate | − | − | − |
| MRS broth (Difco®) supplemented with 0.01% cinnamic acid | − | − | − |

6

```
MRS broth (Difco*) supple-
mented with 0.01%
phenylalanine                               -           -           -

Corn steep based medium
including 4% Corn steep, 5%
lactose, 0.01% sodium pro-
pionate, 0.2% sodium citrate,
1.0% Edamin (enzymatic digest
of lactalbumin) S (Kraft,
Sheffield Products, Kraft,
Inc., Norwich, N.Y.)                        -          +1          +1
```

```
*
            -  = No mycelial masses present.
           +1 = Mycelial masses present greater than 1000.
           +2 = Confluent white lawn.
           +3 = Confluent lawn piled in mass.
           +4 = Confluent lawn pigments green.
```

The above results indicate that the selection of the nutrient medium is important for production of the products (FIC). Even when a whey based medium was supplemented with yeast extract to obtain good Lactobacillus cell growth, sufficient products (FIC) were not produced. Also, the above results indicate that not only are the medium ingredients important for the products (FIC) production, but the brand of a particular ingredient also is important. If Oxoid® yeast extract in the medium was substituted with another brand of yeast extract, Tureen®, expression of the products (FIC) was reduced. The MRS Difco® medium supplemented with 1% Oxoid® yeast extract produced the highest level of the products (FIC) production and thus the best fungal inhibition. Cinnamic acid, phenylalanine and sodium propionate all enhanced production of FIC.

Example 5

Preparation and Production of Dried FIC For Food Application. Lactobacillus casei var. rhamnosus NRRL-B-15972 was grown in broth consisting of the following components: 2% lactose, 1% nonfat dry milk, 1% yeast extract, 0.36% $Na_2HPO_4$, 0.56% $KH_2PO_4$ and trace amounts of $MgSO_4$ and $MnSO_4$. The inorganic salts $Na_2HPO_4$ and $KH_2PO_4$ were made ten (10) times more concentrated than necessary and then added to the growth medium after autoclaving. One (1) liter of the medium was sterilized at 121°C for 15 minutes inoculated with the culture and incubated at 35°C without shaking for 18 hours.

After the culture was grown 18 hours at 35°C in the above medium, the culture flask was then placed in a 70°C incubator for 45 minutes, to heat inactivate the culture. The nutrient medium was then lyophilized 18 hours. From 25 ml of liquid nutrient medium, 1.2 g of dried products (FIC) was obtained.

The titration of the dried products (FIC) with a constant fungal concentration of $10^7$ P. oxalicum spores per ml, as in Example 2, is shown in Table 4.

Table 4

| Amount of dried products (FIC) (g/ml) | | | | | | |
|---|---|---|---|---|---|---|
| Growth Period | 0.3 | 0.4 | 0.6 | 1.2 | 2.4 | Control 0.0 |
| 24 h | - | - | - | - | - | + |
| 48 h | +3 | +3 | +3 | +1 | - | +3 |
| 72 h | +4 | +4 | +4 | +3 | - | +4 |
| - = means no mycellial masses present | | | | | | |
| +1 = visible mycellial masses | | | | | | |
| +2 = white lawn of mycellial masses | | | | | | |
| +3 = white dented lawn of mycellium | | | | | | |
| +4 = final green pigmentation of mycellium | | | | | | |

Titration of 1 g of the dried products (FIC) with a variable number of fungal spores at 25°C is shown in Table 5.

Table 5

| Growth Period | Number (of spores per ml) | | | | Media Control | FIC Control | Fungus Control[a] |
|---|---|---|---|---|---|---|---|
| | $10^7$ | $10^6$ | $10^5$ | $10^4$ | | | |
| 24 h | - | - | - | - | - | - | +1 |
| 48 h | +2 | +2 | +1 | - | - | - | +2 |
| 72 h | +4 | +4 | +4 | +1 | - | - | +4 |
| +1 = visible mycelial masses | | | | | | | |
| +2 = white lawn of mycelial masses | | | | | | | |
| +3 = white dented lawn of mycelium | | | | | | | |
| +4 = final green pigmentation of mycelium | | | | | | | |

(a) $10^7$ spores per ml

The above results indicate that a dried non-extracted FIC preparation exhibits fungal inhibition.

Example 6

Use of Dried Products (FIC) to Extend the Shelf Life of Cottage cheese.

The products (FIC) of Example 5 was sprayed onto surface of Cottage cheese and $10^5$ P. oxalicum spores per ml were added to the Cottage cheese. The products (FIC) were used in a concentration of 1.0 gram per ml. About 35 grams of Cottage cheese were used per test. The tests were conducted at 25°C. The results are shown in Table 6.

Table 6

| Growth Period | Cottage cheese without FIC and added fungus | Cottage cheese with added fungus only | Cottage cheese with FIC only | Cottage cheese with added fungus and Dried FIC |
|---|---|---|---|---|
| 24 h | - | - | - | - |
| 48 h | - | - | - | - |
| 72 h | - | - | - | - |
| 96 h | - | +4 | - | - |
| - = no mycellial masses present. | | | | |
| +4 = final green pigmentation of mycelium, not edible. | | | | |

The products (FIC) of the present invention will inhibit up to $10^7$ spores per ml of Penicillium oxalicum for at least 24 hours in the least concentrated form. The products (FIC) will easily inhibit up to $10^3$ spores per ml which is a very significant mold population in a material. A mold population of greater than 10 per ml is significant.

## Claims

1. A process for producing yeast and mold inhibiting products (FIC) which comprises:
   (a) incubating live cells of a Lactobacillus species in a nutrient medium for the cells containing a sulfur containing organic compound which induces the formation of the products (FIC) and a carbon source so as to produce an isolatable amount of the products (FIC) in the nutrient medium, wherein the products (FIC) inhibit Penicillium oxalicum spores in an assay with the products (FIC) and the Penicillium oxalicum spores mixed.

2. A process for producing yeast and mold inhibiting products (FIC) which comprises:
   (a) incubating suitable live cells of a Lactobacillus species in a nutrient medium for the cells containing growth factors present in cysteine, garlic extract, milk, whey, yeast, yeast extract, molasses or protein digest which induce the formation of the products (FIC), a protein source and a carbon source, so as to produce the products (FIC) in the nutrient medium; and
   (b) treating the growth medium which has been incubated so as to produce the products (FIC) with or without the live cells, wherein the products (FIC) inhibit Penicillium oxalicum spores in an assay with the products (FIC) and the Penicillium oxalicum spores mixed.

3. The process of claim 2 wherein the nutrient medium which is incubated contains a yeast extract for the growth factors, and non-fat dry milk as a protein source; corn steep as a source of essential minerals and lactose, sucrose, dextrose, fructose or molasses as the carbon source.

4. The process of claim 3 wherein the nutrient medium contains a compound selected from the group consisting of cinnamic acid, an alkali metal propionate and phenylalanine, in amounts which increase the production of the products (FIC).

5. The process of claim 2 wherein after the nutrient medium is incubated, the growth medium with the live cells is dried.

6. The products (FIC) of the process of claim 1.

7. A process for producing yeast and mold inhibiting products (FIC) which comprises:
   (a) incubating suitable live cells of a Lactobacillus species in a nutrient medium for the cells containing growth factors present in cysteine, garlic extract, milk, whey, yeast, yeast extract, molasses or protein digest which induce the formation of the products (FIC), a protein source and a carbon source so as to produce products (FIC) in the nutrient medium wherein the products (FIC) inhibit Penicillium oxalicum spores in an assay with the products (FIC) and the Penicillium oxalicum spores mixed; and

(b) treating the nutrient medium which has been incubated so as to produce the products (FIC) with the cells disrupted, wherein the products (FIC) inhibit Penicillium oxalicum spores in an assay with the products (FIC) and the Penicillium oxalicum spores mixed.

8. The process of claim 7 wherein after the nutrient medium is incubated, the nutrient medium with the live cells of Lactobacillus is dried.

9. The process of claim 7 wherein the Lactobacillus are incubated in the nutrient medium at a temperature of between about 10º and 50ºC and then dried to produce a concentrate of the products (FIC).

10. The process of claim 7 wherein the nutrient medium contains yeast extract, whey or non-fat dry milk as a protein source, corn steep as a source of essential salts and lactose, sucrose, dextrose, fructose or molasses as the carbon source.

11. The process of claim 10 wherein the nutrient medium contains a manganese salt and a magnesium salt as essential salts and an alkali metal phosphate as a buffer.

12. The products (FIC) of the process of claim 7.

13. A process for producing yeast and mold inhibiting products (FIC) which comprises:
(a) incubating suitable cells of a Lactobacillus species in a nutrient medium for the cells containing growth factors present in cysteine, garlic extract, milk, whey, yeast, yeast extract, molasses or protein digest which induce the formation of the products (FIC) and a carbon source so as to produce the products (FIC) in the nutrient medium wherein the products (FIC) inhibit Penicillium oxalicum spores in an assay with the products (FIC) and the Penicillium oxalicum spores mixed;
(b) mixing the incubated nutrient medium with a water immiscible organic solvent which extracts the product into the solvent;
(c) separating the solvent with the extracted product from the nutrient medium; and
(d) separating the products (FIC) from the solvent.

14. The process of claim 13 wherein the solvent is n-butanol.

15. The process of claim 13 wherein the solvent is acetone.

16. The process of claim 13 wherein the solvent is ethanol.

17. The process of claim 13 wherein the solvent mixture is cooled to thereby precipitate impurities.

18. The process of claim 13 wherein in addition the separated products are purified to separate impurities using liquid chromatography.

19. The products (FIC) produced by the process of claim 13, wherein the products (FIC) have a primary ultraviolet absorbance at about 269 nanometers.

20. The process of claim 13 wherein in addition the products (FIC) are purified by liquid dialysis which passes compounds through a membrane having a molecular size of about 1000 or less and wherein the products pass through the membrane and are collected.

21. The purified products (FIC) of the process of claim 20.

22. The process of claim 20 wherein in addition the purified products (FIC) are further purified by liquid chromatography using a solvent mixture to produce separation of the products (FIC) from impurities.

23. The process of claim 22 wherein in addition the products (FIC) without the impurities are repurified using liquid chromatography.

24. The process of claim 13 wherein the nutrient medium which is incubated contains growth promoting amounts of a sulfur containing organic compound, yeast extract and protein digest.

10

25. The process of claim 1 wherein the nutrient medium which is to be incubated is pasteurized or sterilized at about 100°C or above prior to incubating the cells.

26. A method for preventing yeast and mold growth in a material in need thereof which comprises: adding to the material products (FIC) produced by a process which comprises incubating suitable live cells of a Lactobacillus in a nutrient medium containing a sulfur containing organic compound, a protein source and a carbon source, which induce the formation of the yeast and mold inhibiting products (FIC) in the nutrient medium wherein the products (FIC) in the nutrient medium inhibit Penicillium oxalicum spores in an assay with the products (FIC) and the Penicillium oxalicum spores mixed and then treating the nutrient medium which now contains live cells, in such a manner so as to produce the final products (FIC) in a form containing live cells, dead cells or no cells to thereby prevent yeast and mold growth in the material.

27. The method of claim 26 wherein the material is a food.

28. The method of claim 27 wherein the food is a carbonated beverage, Cottage cheese, yogurt, margarine, bread, grains and nuts.

29. The method of claim 26 wherein the material is living tissue.

30. The method of claim 27 wherein the material is a cleanser.

31. A process for producing yeast and mold inhibiting products (FIC) which comprises:
(a) incubating suitable cells of a Lactobacillus species in a nutrient medium for the cells containing growth factors present in cysteine, garlic extract, milk, whey, yeast, yeast extract, molasses or protein digest which induce the formation of the products (FIC), a protein source and a carbon source so as to produce the products (FIC) in the nutrient medium wherein the products (FIC) inhibit Penicillium oxalicum spores in an assay with the products (FIC) and the Penicillium oxalicum spores mixed; and
(b) separating the products (FIC) from the nutrient medium and cells.

32. A process for producing yeast and mold inhibiting products (FIC) which comprises:
(a) incubating suitable live cells of a Lactobacillus species in a nutrient medium for the cells so as to produce an isolatable amount of the products (FIC) in the nutrient medium, wherein the products (FIC) separated from the medium inhibit Penicillium oxalicum up to a level of about $10^7$ spores per ml when mixed in an agar-broth medium.

33. A composition containing the products (FIC) produced by the process of claim 32.

**Patentansprüche**

1. Verfahren zum Herstellen von Hefe- und Schimmelpilz-hemmenden Produkten (fungal inhibiting compounds = FIC), umfassend:
(a) Inkubieren von lebenden Zellen einer Lactobacillus-Art in einem Nährmedium für die Zellen, enthaltend eine Schwefel enthaltende organische Verbindung, die die Bildung der Produkte (FIC) induziert und eine Kohlenstoffquelle, um eine isolierbare Menge der Produkte (FIC) in dem Nährmedium herzustellen, worin die Produkte (FIC) Penicillium oxalicum-Sporen in einem Ansatz hemmen, in dem die Produkte (FIC) und die Penicillium oxalicum-Sporen gemischt sind.

2. Verfahren zum Herstellen von Hefe- und Schimmelpilz-hemmenden Produkten (FIC), umfassend:
(a) Inkubieren geeigneter lebender Zellen einer Lactobacillus-Art in einem Nährmedium für die Zellen, enthaltend Wachstumsfaktoren, die in Cystein, Knoblauchextrakt, Milch, Molke, Hefe, Hefeextrakt, Melasse oder Proteinabbauprodukten vorhanden sind, welche die Bildung der Produkte (FIC) induzieren, eine Proteinquelle und eine Kohlenstoffquelle, um die Produkte (FIC) in dem Nährmedium herzustellen; und
(b) Behandeln des Wachstumsmediums, welches inkubiert worden ist, um die Produkte (FIC) mit oder ohne lebende Zellen herzustellen, worin die Produkte (FIC) Penicillium oxalicum-Sporen in einem Ansatz hemmen, in dem die Produkte (FIC) und die Penicillium oxalicum-Sporen gemischt sind.

**3.** Verfahren nach Anspruch 2, worin das Nährmedium, welches inkubiert wird, einen Hefeextrakt für die Wachstumsfaktoren und fettfreie Trockenmilch als Proteinquelle, gewässertes Getreide als Quelle essentieller Mineralien und Lactose, Saccharose, Dextrose, Fructose oder Melasse als Kohlenstoffquelle enthält.

**4.** Verfahren nach Anspruch 3, worin das Nährmedium eine Verbindung, ausgewählt aus der Gruppe, bestehend aus Zimtsäure, einem Alkalimetallpropionat und Phenylalanin in Mengen enthält, die die Herstellung der Produkte (FIC) steigert.

**5.** Verfahren nach Anspruch 2, worin das Wachstumsmedium mit den lebenden Zellen getrocknet wird, nachdem das Nährmedium inkubiert worden ist.

**6.** Produkte (FIC) des Verfahrens nach Anspruch 1.

**7.** Verfahren zum Herstellen von Hefe- und Schimmelpilzhemmenden Produkten (FIC), umfassend:
(a) Inkubieren geeigneter lebender Zellen einer Lactobacillus-Art in einem Nährmedium für die Zellen, enthaltend Wachstumsfaktoren, die in Cystein, Knoblauchextrakt, Milch, Molke, Hefe, Hefeextrakt, Melasse oder Proteinabbauprodukten vorhanden sind, welche die Bildung der Produkte (FIC) induzieren, eine Proteinquelle und eine Kohlenstoffquelle, um die Produkte (FIC) in dem Nährmedium herzustellen, worin die Produkte (FIC) Penicillium oxalicum-Sporen in einem Ansatz hemmen, in dem die Produkte (FIC) und die Penicillium oxalicum-Sporen gemischt sind; und
(b) Behandeln des Nährmediums, welches inkubiert worden ist, um die Produkte mit den aufgebrochenen Zellen herzustellen, worin die Produkte (FIC) Penicillium oxalicum-Sporen in einem Ansatz hemmen, in dem die Produkte (FIC) und die Penicillium oxalicum-Sporen gemischt sind.

**8.** Verfahren nach Anspruch 7, worin das Nährmedium mit den lebenden Lactobacillus-Zellen getrocknet wird, nachdem das Nährmedium inkubiert worden ist.

**9.** Verfahren nach Anspruch 7, worin die Lactobacillus-Zellen in dem Nährmedium mit einer Temperatur zwischen ungefähr 10° und 50° C inkubiert und danach getrocknet werden, um ein Konzentrat der Produkte (FIC) herzustellen.

**10.** Verfahren nach Anspruch 7, worin das Nährmedium Hefeextrakt, Molke oder fettfreie Trockenmilch als Proteinquelle, gewässertes Getreide als Quelle essentieller Salze und Lactose, Saccharose, Dextrose, Fructose oder Melasse als Kohlenstoffquelle enthält.

**11.** Verfahren nach Anspruch 10, worin das Nährmedium ein Mangansalz und ein Magnesiumsalz als essentielle Salze und ein Alkalimetallphosphat als Puffer enthält.

**12.** Produkte (FIC) des Verfahrens nach Anspruch 7.

**13.** Verfahren zum Herstellen von Hefe- und Schimmelpilzhemmenden Produkten (FIC), umfassend:
(a) Inkubieren geeigneter Zellen einer Lactobacillus-Art in einem Nährmedium für die Zellen, enthaltend Wachstumsfaktoren, die in Cystein, Knoblauchextrakt, Milch, Molke, Hefe, Hefeextrakt, Melasse oder Proteinabbauprodukten vorhanden sind, welche die Bildung der Produkte (FIC) induzieren, und eine Kohlenstoffquelle, um die Produkte (FIC) in dem Nährmedium herzustellen, worin die Produkte (FIC) Penicillium oxalicum-Sporen in einem Ansatz hemmen, in dem die Produkte (FIC) und die Penicillium oxalicum-Sporen gemischt sind;
(b) Mischen des inkubierten Nährmediums mit einem mit Wasser unmischbaren organischen Lösungsmittel, welches das Produkt in das Lösungsmittel extrahiert;
(c) Trennen des Lösungsmittels mit dem extrahierten Produkt von dem Nährmedium; und
(d) Trennen der Produkte (FIC) von dem Lösungsmittel.

**14.** Verfahren nach Anspruch 13, worin das Lösungsmittel n-Butanol ist.

**15.** Verfahren nach Anspruch 13, worin das Lösungsmittel Aceton ist.

12

**16.** Verfahren nach Anspruch 13, worin das Lösungsmittel Ethanol ist.

**17.** Verfahren nach Anspruch 13, worin die Lösungsmittelmischung gekühlt wird, um dadurch Verunreinigungen zu präzipitieren.

**18.** Verfahren nach Anspruch 13, worin zusätzlich die abgetrennten Produkte unter Verwendung von Flüssigchromatographie gereinigt werden, um Verunreinigungen abzutrennen.

**19.** Produkte (FIC), hergestellt durch das Verfahren des Anspruchs 13, worin die Produkte (FIC) eine primäre Ultraviolettabsorption bei ungefähr 269 Nanometern haben.

**20.** Verfahren nach Anspruch 13, worin zusätzlich die Produkte (FIC) durch Flüssigdialyse gereinigt werden, bei welcher Verbindungen mit einer Molekulargröße von ungefähr 1000 oder weniger durch eine Membran passieren und worin die Produkte durch die Membran passieren und gesammelt werden.

**21.** Gereinigte Produkte (FIC) des Verfahrens nach Anspruch 20.

**22.** Verfahren nach Anspruch 20, worin zusätzlich die gereinigten Produkte (FIC) durch Flüssigchromatographie unter Verwendung einer Lösungsmittelmischung weiter gereinigt werden, um die Trennung der Produkte (FIC) von Verunreinigungen herzustellen.

**23.** Verfahren nach Anspruch 22, worin zusätzlich die Produkte (FIC) ohne Verunreinigung nochmals unter Verwendung von Flüssigchromatographie gereinigt werden.

**24.** Verfahren nach Anspruch 13, worin das Nährmedium, welches inkubiert wird, wachstumsfördernde Mengen einer Schwefel-enthaltenden organischen Verbindung, Hefeextrakt und Proteinabbauprodukte enthält.

**25.** Verfahren nach Anspruch 1, worin das Nährmedium, welches inkubiert werden soll, bei ungefähr 100° C oder darüber vor dem Inkubieren der Zellen pasteurisiert oder sterilisiert ist.

**26.** Verfahren zum Verhindern von Hefe- und Schimmelpilzwachstum in einem Material, welches umfaßt: Zufügen der Produkte (FIC) zu dem Material, die hergestellt sind durch ein Verfahren, welches umfaßt: Inkubieren geeigneter lebender Zellen einer Lactobacillus-Art in einem Nährmedium, enthaltend eine Schwefel-enthaltende organische Verbindung, eine Proteinquelle und eine Kohlenstoffquelle, welche die Bildung der Hefe- und Schimmelpilz-hemmenden Produkte (FIC) in dem Nährmedium induziert, worin die Produkte (FIC) in dem Nährmedium Penicillium oxalicum-Sporen in einem Ansatz hemmen, in dem die Produkte (FIC) und die Penicillium oxalicum-Sporen gemischt sind und danach Behandeln des Nährmediums, welches jetzt lebende Zellen enthält, auf eine Weise, um die endgültigen Produkte (FIC) in einer Form, enthaltend lebende Zellen, tote Zellen oder keine Zellen herzustellen, um dadurch Hefe und Schimmelpilzwachstum in dem Material zu verhindern.

**27.** Verfahren nach Anspruch 26, worin das Material ein Lebensmittel ist.

**28.** Verfahren nach Anspruch 27, worin das Lebensmittel ein Kohlensäure-haltiges Getränk, Hüttenkäse, Yoghurt, Margarine, Brot, Getreide oder Nüsse ist.

**29.** Verfahren nach Anspruch 26, worin das Material lebendes Gewebe ist.

**30.** Verfahren nach Anspruch 27, worin das Material ein Reinigungsmittel ist.

**31.** Verfahren zum Herstellen von Hefe- und Schimmelpilz-hemmenden Produkten (FIC), umfassend:
(a) Inkubieren geeigneter Zellen einer Lactobacillus-Art in einem Nährmedium für die Zellen, enthaltend Wachstsumsfaktoren, die in Cystein, Knoblauchextrakt, Milch, Molke, Hefe, Hefeextrakt, Melasse oder Proteinabbauprodukten vorhanden sind, welche die Bildung der Produkte (FIC) induzieren, eine Proteinquelle und eine Kohlenstoffquelle, um die Produkte (FIC) in dem Nährmedium herzustellen, worin die Produkte (FIC) Penicillium oxalicum-Sporen in einem Ansatz hemmen, in dem die Produkte (FIC) und die Penicillium oxalicum-Sporen gemischt sind; und

(b) Trennen der Produkte (FIC) von dem Nährmedium und Zellen.

**32.** Verfahren zum Herstellen von Hefe- und Schimmelpilz-hemmenden Produkten (FIC), umfassend:

(a) Inkubieren geeigneter lebender Zellen einer Lactobacillus-Art in einem Nährmedium für die Zellen, um eine isolierbare Menge der Produkte (FIC) in dem Nährmedium herzustellen, worin die von den Medium abgetrennten Produkte (FIC) Penicillium oxalicum bis zu einer Rate von ungefähr $10^7$ Sporen pro ml hemmen, wenn diese in ein Agar-Kulturmedium gemischt sind.

**33.** Zusammensetzung, enthaltend die Produkte (FIC), hergestellt durch das Verfahren des Anspruchs 32.

**Revendications**

**1.** Procédé pour la production de produits inhibiteurs de levures et de moisissures (CIC = composés inhibiteurs de champignons), comprenant:

(a) l'incubation de cellules vivantes d'une espèce appartenant genre Lactobacillus, dans un milieu nutritif pour les cellules, contenant un composé organique soufré qui induit la formation des produits (CIC), et une source de carbone de manière à produire une quantité isolable des produits (CIC) dans le milieu nutritif, les produits (CIC) inhibant des spores de Penicillium oxalicum dans un essai dans lequel sont mélangés les produits (CIC) et les spores de Penicillium oxalicum.

**2.** Procédé pour la production de produits inhibiteurs de levures et de moisissures (CIC), comprenant:

(a) l'incubation de cellules vivantes appropriées, d'une espèce appartenant au genre Lactobacillus, dans un milieu nutritif pour les cellules, contenant des facteurs de croissance présents dans la cystéine, l'extrait d'ail, le lait, le lactosérum, la levure, l'extrait de levure, la mélasse ou un produit de digestion de protéines qui induit la formation des produits (CIC), une source de protéines et une source de carbone, de manière à produire les produits (CIC) dans le milieu nutritif; et

(b) le traitement du milieu de culture qui a été mis à incuber, de manière à produire les produits (CIC) avec ou sans les cellules vivantes, les produits (CIC) inhibant des spores de Penicillium oxalicum dans un essai dans lequel sont mélangés les produits (CIC) et les spores de Penicillium oxalicum.

**3.** Procédé selon la revendication 2, dans lequel le milieu nutritif qui est mis à incuber contient un extrait de levure pour les facteurs de croissance et du lait écrémé en poudre en tant que source de protéines; de l'extrait de liqueur de trempage du maïs en tant que source de minéraux essentiels, et du lactose, du saccharose, du D-glucose, du fructose ou de la mélasse en tant que source de carbone.

**4.** Procédé selon la revendication 3, dans lequel le milieu nutritif contient un composé choisi parmi l'acide cinnamique, un propionate alcalin et la phénylalanine, en quantités qui augmentent la production des produits (CIC).

**5.** Procédé selon la revendication 2, dans lequel, après incubation du milieu nutritif, le milieu de croissance contenant les cellules vivantes est séché.

**6.** Les produits (CIC) du procédé selon la revendication 1.

**7.** Procédé pour la production de produits inhibiteurs de levures et de moisissures (CIC), comprenant:

(a) l'incubation de cellules vivantes appropriées d'une espèce appartenant au genre Lactobacillus, dans un milieu nutritif pour les cellules, contenant des facteurs de croissance présents dans la cystéine, l'extrait d'ail, le lait, le lactosérum, la levure, l'extrait de levure, la mélasse ou un produit de digestion de protéines qui induit la formation des produits (CIC), une source de protéines et une source de carbone, de manière à produire les produits (CIC) dans le milieu nutritif; les produits (CIC) inhibant des spores de Penicillium oxalicum dans un essai dans lequel sont mélangés les produits (CIC) et les spores de Penicillium oxalicum; et

(b) le traitement du milieu nutritif qui a été mis à incuber, de manière à produire les produits (CIC) avec les cellules lysées, les produits (CIC) inhibant des spores de Penicillium oxalicum dans un essai dans lequel sont mélangés les produits (CIC) et les spores de Penicillium oxalicum.

**8.** Procédé selon la revendication 7, dans lequel, après incubation du milieu nutritif, le milieu nutritif contenant les cellules vivantes de Lactobacillus est séché.

**9.** Procédé selon la revendication 7, dans lequel les Lactobacillus sont mis à incuber dans le milieu nutritif à une température comprise entre environ 10 et 50°C, et ensuite séchés pour la production d'un concentré des produits (CIC).

**10.** Procédé selon la revendication 7, dans lequel le milieu nutritif contient de l'extrait de levure, du lactosérum ou du lait écrémé en poudre, en tant que source de protéines, de l'extrait de liqueur de trempage du maïs en tant que source de sels essentiels et du lactose, du saccharose, du D-glucose, du fructose ou de la mélasse en tant que source de carbone.

**11.** Procédé selon la revendication 10, dans lequel le milieu nutritif contient un sel de manganèse et un sel de magnésium en tant que sels essentiels, et un phosphate de métal alcalin en tant que tampon.

**12.** Les produits (CIC) du procédé de la revendication 7.

**13.** Procédé pour la production de produits inhibiteurs de levures et de moisissures (CIC), comprenant:
(a) l'incubation de cellules appropriées d'une espèce appartenant au genre Lactobacillus, dans un milieu nutritif pour les cellules, contenant des facteurs de croissance présents dans la cystéine, l'extrait d'ail, le lait, le lactosérum, la levure, l'extrait de levure, la mélasse ou un produit de digestion de protéines qui induit la formation des produits (CIC) et une source de carbone, de manière à produire les produits (CIC) dans le milieu nutritif; les produits (CIC) inhibant des spores de Penicillium oxalicum dans un essai dans lequel sont mélangés les produits (CIC) et les spores de Penicillium oxalicum;
(b) le mélangeage du milieu nutritif, qui a été mis à incuber, avec un solvant organique non miscible à l'eau, qui extrait le produit dans le solvant;
(c) la séparation du solvant, contenant le produit extrait, d'avec le milieu nutritif; et
(d) la séparation des produits (CIC) d'avec le solvant.

**14.** Procédé selon la revendication 13, dans lequel le solvant est le n-butanol.

**15.** Procédé selon la revendication 13, dans lequel le solvant est l'acétone.

**16.** Procédé selon la revendication 13, dans lequel le solvant est l'éthanol.

**17.** Procédé selon la revendication 13, dans lequel on refroidit le mélange à base de solvant, pour faire ainsi précipiter les impuretés.

**18.** Procédé selon la revendication 13, dans lequel on purifie en outre les produits séparés, pour séparer les impuretés au moyen de chromatographie liquide.

**19.** Les produits (CIC) produits par le procédé de la revendication 13, les produits (CIC) ayant une absorption primaire dans l'ultraviolet à environ 269 nm.

**20.** Procédé selon la revendication 13, dans lequel les produits (CIC) sont en outre purifiés par dialyse de liquide laissant passer les composés à travers une membrane ayant une taille moléculaire d'environ 1 000 ou moins, et dans lequel les produits traversant la membrane sont recueillis.

**21.** Les produits (CIC) purifiés du procédé de la revendication 20.

**22.** Procédé selon la revendication 20, dans lequel, en outre, les produits (CIC) purifiés sont purifiés davantage par chromatographie liquide au moyen d'un mélange à base de solvant, pour aboutir a la séparation des produits (CIC) d'avec les impuretés.

**23.** Procédé selon la revendication 22, dans lequel, en outre, les produits (CIC) sans les impuretés sont repurifiés au moyen de chromatographie liquide.

**24.** Procédé selon la revendication 13, dans lequel le milieu nutritif qui est mis à incuber contient des quantités, stimulant la croissance, d'un composé organique soufré, d'un extrait de levure et d'un produit de digestion de protéines.

**25.** Procédé selon la revendication 1, dans lequel le milieu nutritif qui est destiné à être mis à incuber est pasteurisé ou stérilisé aux environs de 100°C ou au-dessus, avant l'incubation des cellules.

**26.** Procédé pour la prévention de la croissance de levures ou de moisissures, dans une matière nécessitant un tel traitement, comprenant:
l'addition à la matière de produits (CIC) produits par un procédé comprenant l'incubation de cellules vivantes appropriées d'un Lactobacillus dans un milieu nutritif contenant un composé organique soufré, une source de protéines et une source de carbone, qui induit la formation des produits (FIC), dans le milieu nutritif, les produits (CIC) inhibant des spores de Penicillium oxalicum dans un essai dans lequel sont mélangés les produits (CIC) et les spores de Penicillium oxalicum,
et ensuite le traitement du milieu nutritif qui contient désormais des cellules vivantes, de manière à produire les produits (CIC) finals sous une forme contenant des cellules vivantes, des cellules mortes ou ne contenant pas de cellules, pour empêcher ainsi la croissance de levures et de moisissures dans la matière.

**27.** Procédé selon la revendication 26, dans lequel la matière est un aliment.

**28.** Procédé selon la revendication 27, dans lequel l'aliment est une boisson gazeuse, du fromage blanc, du yogourt, de la margarine, du pain, des graines et des fruits à écales.

**29.** Procédé selon la revendication 27, dans lequel la matière est un tissu vivant.

**30.** Procédé selon la revendication 26, dans lequel la matière est un produit de nettoyage.

**31.** Procédé pour la production de produits inhibiteurs de levures et de moisissures (CIC), comprenant:
(a) l'incubation de cellules appropriées d'une espèce appartenant au genre Lactobacillus, dans un milieu nutritif pour les cellules, contenant des facteurs de croissance présents dans la cystéine, l'extrait d'ail, le lait, le lactosérum, la levure, l'extrait de levure, la mélasse ou un produit de digestion de protéines qui induit la formation des produits (CIC), et une source de protéines et une source de carbone, de manière à produire les produits (CIC) dans le milieu nutritif; les produits (CIC) inhibant des spores de Penicillium oxalicum dans un essai dans lequel sont mélangés les produits (CIC) et les spores de Penicillium oxalicum; et
(b) la séparation des produits (CIC) d'avec le milieu nutritif et les cellules.

**32.** Procédé pour la production de produits inhibiteurs de levures et de moisissures, comprenant:
(a) l'incubation de cellules vivantes appropriées d'une espèce appartenant au genre Lactobacillus, dans un milieu nutritif pour les cellules, de manière à produire une quantité isolable des produits (CIC) dans le milieu nutritif, les produits (CIC) séparés du milieu inhibant Penicillium oxalicum jusqu'à une quantité d'environ $10^7$ spores par ml, lorsqu'ils sont mélangés dans un milieu de type bouillon gélosé.

**33.** Composition contenant les produits (CIC) produits par le procédé selon la revendication 32.

16